(19) Europäisches Patentamt
European Patent Office
Office européen des brevets

(11) Publication number: 0 288 427 A2

(12) # EUROPEAN PATENT APPLICATION

(21) Application number: 88810103.7

(22) Date of filing: 22.02.88

(51) Int. Cl.[4]: C 07 C 120/00
C 07 C 120/06, C 07 C 121/75

(30) Priority: 24.02.87 US 18305

(43) Date of publication of application:
26.10.88 Bulletin 88/43

(84) Designated Contracting States:
AT BE CH DE ES FR GB GR IT LI LU NL SE

(71) Applicant: SANDOZ AG
Lichtstrasse 35
CH-4002 Basel (CH)

(84) Designated Contracting States:
BE CH ES FR GB GR IT LI LU NL SE

(71) Applicant: SANDOZ-PATENT-GMBH
Humboldtstrasse 3
D-7850 Lörrach (DE)

(84) Designated Contracting States: DE

(71) Applicant: SANDOZ-ERFINDUNGEN
Verwaltungsgesellschaft m.b.H.
Brunner Strasse 59
A-1235 Wien (AT)

(84) Designated Contracting States: AT

(72) Inventor: Gruber, John Myron
565 Willow Road, Apt. 4
Menlo Park, CA 94025 (US)

The title of the invention has been amended (Guidelines for Examination in the EPO, A-III, 7.3).

(54) **Process for the preparation of alpha-cyano esters.**

(57) A process for the preparation of α-cyanobenzyl esters of cyclopropane carboxylic acids and substituted alkanoic acids by treating a carboxylic acid and a benzaldehyde with a metal cyanide, followed by treatment with a sulfonyl halide.

EP 0 288 427 A2

**Description**

## IMPROVEMENTS IN OR RELATING TO ORGANIC COMPOUNDS

This invention relates to an improved process for the preparation of α-cyanobenzyl esters of cyclopropane carboxylic acids and substituted alkanoic acids, which esters are active pesticides and form part of the class of compounds known as synthetic pyrethroids.

More particularly, the present invention provides a one-pot, two-reaction process for the preparation of an α-cyano ester of formula I

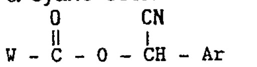 I

wherein W is an optionally substituted cycloalkyl or substituted alkyl group containing up to 24 carbon atoms, and Ar is a substituted aromatic or heteroaromatic group, which process comprises

a) treating a carboxylic acid of formula W-COOH and an aldehyde of formula II

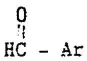 II

with an alkali or alkaline earth metal cyanide in an acid acceptor solvent which is optionally diluted by an aprotic solvent, and

b) treating the resulting mixture with a sulfonyl halide.

When W represents an optionally-substituted cycloalkyl group in formula I, the preferred compounds are those containing a cyclopropyl group of formula III

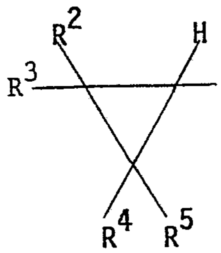 III

wherein,

each of $R^2$ and $R^3$ is independently $C_{1-6}$alkyl or halogen; or $R^2$ represents hydrogen, and $R^3$ represents $C_{1-6}$alkyl, $C_{1-6}$haloalkyl, $C_{2-6}$alkenyl or $C_{2-6}$haloalkenyl; and

each of $R^4$ and $R^5$ is independently $C_{1-6}$alkyl or $R^4$ is hydrogen and $R^5$ is $C_{1-6}$alkyl, $C_{1-6}$haloalkyl, $C_{2-6}$alkenyl or $C_{1-6}$haloalkenyl.

When W represents an optionally substituted benzyl group in formula I, preferred compounds are those containing a substituted benzyl group of formula IV

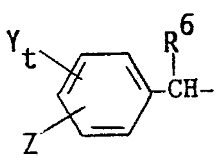 IV

wherein,

$R^6$ is $C_{1-6}$alkyl or cyclopropyl;

t is zero, one or two; and

each of Y and Z is independently hydrogen, $C_{1-6}$alkyl, $C_{1-6}$haloalkyl, $C_{1-6}$alkoxy, $C_{1-6}$haloalkoxy, halogen or nitro.

When W represents an optionally substituted anilinoalkyl group in formula I, preferred compounds are those of formula V

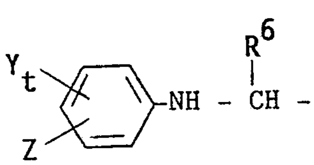 V

wherein, the values of $R^6$, t, Y and Z are as defined hereinabove.

In the practice of the present invention, $R^2$ is preferably methyl or chloro.

$R^3$ is preferably methyl, chloro, isobutenyl, mono- or dichlorovinyl, or dibromovinyl, 1,2,2,2-tetrabrom-ethyl, or 1-(2-chloro-3,3,3-trifluoro-1-propenyl).

$R^4$ is preferably methyl.

$R^5$ is preferably methyl, isobutenyl or mono- or dichlorovinyl.

$R^6$ is preferably a branched chain alkyl such as isopropyl.

t is preferably zero or one.

Y is preferably $CF_3$.

Z is preferably methyl, methoxy or chloro.

Ar is preferably a substituted phenyl group and more preferably a group of formula VI

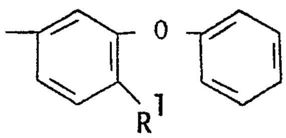

VI

wherein $R^1$ is hydrogen or fluoro.

Many synthetic pyrethroid compounds of formula I are known in the art and are disclosed in, for example, U.S. Patent Nos. 3,835,176; 3,973,035; 3,973,036; 3, 987,193; 3,996,244; 4,100,298; 4,411,912 and 4,260,633.

The preferred α-cyano-3-phenoxybenzyl pyrethroids for use as pesticides are of the formula I where $R^1$ is hydrogen and W is 1-(2-chloro-4-trifluoromethylanilino)-2-methylpropyl, α-isopropyl-4-chlorobenzyl, 2,2,3,3-tetramethylcyclopropyl, 2-(2,2-dichlorovinyl)-3,3-dimethylcyclopropyl or 2-(2,2-dibromovinyl)-3,3-dimethylcyclpropyl, preferably 1-(2-chloro-4-trifluoromethylanilino)-2-methylpropyl.

For carrying out the process of the present invention, from 0.8 to 1.5 moles, preferably about 1.0 to 1.3 moles, of benzaldehyde (II) are employed per mole of carboxylic acid W-COOH.

The alkali or alkali earth metal cyanides are preferably chosen from the alkali metal cyanides such as sodium cyanide and potassium cyanide. Because of its availability and low cost, sodium cyanide is especially preferred. The cyanide salt is generally employed in at least equimolar amount, more generally in excess molar amount, the molar ratio of the cyanide salt to the aldehyde lying in the range of 1:1 to 1.6:1, preferably about 1:1 to 1.3:1.

Suitable examples of acid acceptor solvents are optionally substituted pyridine and tertiary amines.

Where the acid acceptor solvent is substituted pyridine, it is e.g. mono-, di- or tri-substituted; examples of such pyridine substituents are $C_{1-4}$alkyl groups such as methyl and ethyl. Examples of substituted pyridines suitable for use as acid acceptor solvents in the reaction a) of the process of the invention are picolines (2-, 3- or 4-methylpyridine), 5-ethyl-2-methylpyridine, 2,4,6-collidine or mixtures of such substituted pyridines.

Where the acid acceptor solvent is a tertiary amine, it is e.g. a tri(alkyl)amine or N,N-di(alkyl)aniline, in which the alkyl groups have in particular 1 to 4 carbon atoms.

Particularly good results are achieved when employing a picoline or a picoline mixture (e.g. a mixture of 3-methyl- and 4-methyl pyridine) as an acid acceptor solvent.

Aprotic solvents may optionally be and are preferably used in combination with the acid acceptor solvent. In such case, the acid acceptor solvent is conveniently employed in at least equimolar, more preferably in excess molar amount of the carboxylic acid of formula W-COOH employed in process step a).

Suitable examples of aprotic solvents are aromatic hydrocarbons such as benzene, toluent, a xylene, aliphatic hydrocarbons such as hexane, ethers such as tetrahydrofuran, amides of carboxylic acids (including lactams) such as N,N-dimethylformamide and N-methylpyrrolidinone and chlorinated hydrocarbons such as methylenechloride, or mixtures of such aprotic solvent. Among these, toluene and/or N-methylpyrrolidinone are preferred.

An esterification catalyst such as 4-dimethylaminopyridine may optionally be employed in step b) of the process.

The sulfonyl halides utilized in step b) of the present invention are conveniently selected from the $C_{1-4}$alkanesulfonyl halides, such as methanesulfonyl chloride and ethanesulfonyl bromide, and the arylsulfonyl halides, such as benzenesulphonyl chloride and toluenesulfonyl chloride. Due to its low cost and ready availability, methanesulfonyl chloride is preferred. The sulfonyl halide is generally employed in an excess molar amount, the molar ratio of the sulfonyl halide to the carboxylic acid lying in the range 1:1 to 1.5:1, preferably about 1.1:1 to 1.2:1.

Step a) of the process of the present invention is carried out by mixing together all ingredients but the sulfonyl halide, maintaining the temperature of the reactants between 0° and 80° C, preferably between about 50° and 60° C, and stirring the reactants until the reaction is complete. In this reaction the carboxylic acid salt and the cyanohydrin of the aldehyde are formed.

This is followed immediately by step b) wherein the reaction mixture is then cooled to between 0° and 50° C, preferably to about 0° to 20°, and the sulfonyl halide is slowly added while maintaining a stable reaction temperature until the reaction is complete.

The compounds of formula I may be recovered from the reaction mixture in which they are formed by working up by established procedures.

The α-cyanobenzyl esters prepared by the process according to the present invention can exist in a number of steroisomeric forms, and therefore the present invention includes the production of any one of a mixture of such steroisomers. The required stereoisomer or mixture of stereoisomers may be obtained by using as starting material the appropriate stereoisomeric carboxylic acid. This process produces a 1:1 mixture of stereoisomers at the α-cyano carbon.

The following examples are provided to illustrate the practice of the present invention. Temperature is given in degrees Centigrade.

**EXAMPLE 1**

A mixture of sodium cyanide (134.75 g, 2.75 mol), picolines ("mixed" picolines, obtained from Reilly Tar &; Chemical Co., comprising 97% 3- and 4-methylpyridine) (418.50 g, 4.50 mol), 3-phenoxybenzaldehyde (519.75 g, 2.54 mol), (R)-2-(2-chloro-4-tri-fluoromethylanilino)-3-methylbutanoic acid (738.37 g, 2.50 mol) in N-methylpyrrolidinone (428 g, 4.3 mol) and 3750 ml of toluene is stirred for 3 hours, maintaining the reaction at 55°. The reaction mixture is then cooled to 7° with an ice bath, and methanesulfonyl chloride (327.75 g, 2.87 mol) is added slowly over 1 hours, while maintaining an internal temperature of 15°. The mixture is stirred at 15° for an additional 6 hours to complete esterification. (The completion of esterification is judged by GLPC on the disappearance of cyanohydrin.)

To the reaction mixture is added a solution of potassium hydroxide (241.0 g, 4.3 mol) in 2500 ml of water. After the solids have dissolved, the phases are separated and the organic phase is again washed with aqueous potassium hydroxide, with aqueous hydrochloric acid (2x) and then with water. The solvent is stripped to give, as an amber coloured oil, (R,S)-α-cyano-3-phenoxybenzyl (R)-2-(2-chloro-4-trifluoromethylanilino)-3-methylbutanoate, in 90.48% yield (based on the weight of the starting butanoic acid).

**EXAMPLE 2**

Following the procedure of Example 1, to a mixture of 3-(2,2-dichlorovinyl)-2,2-dimethylcyclopropane-1-carboxylic acid (50 mmol), 3-phenoxy-benzaldehyde (9.90 g, 50 mmol), N-methylpyrrolidone (10 g) and picolines (8.5 g) in toluene is added sodium cyanide (2.57 g, 52.2 mmol), followed ca. 3 hours later by slow addition of methanesulfonyl chloride (6.87 g, 60 mmol). The reaction mixture is worked up and the solvent is removed to give α-cyano-3-phenoxybenzyl 3-(2,2-dichlorovinyl)-2,2-dimethylcyclopropanecarboxylate.

**EXAMPLE 3**

A mixture of 4-(dimethylamino)pyridine (0.50 g, 0.0041 mol), sodium cyanide (13.50 g, 0.275 mol), 3-phenoxybenzaldehyde (51.05 g, 0.250 mol), (R)-2-(2-chloro-4-trifluoromethylanilino)-3-methylbutanoic acid (71.58 g, 0.242 mol) in N-methylpyrrolidinone (44.3 g, 0.44 mol) and picolines ("mixed", obtained from Reilly Tar &; Chemical Co.) (42.5 g, 0.455 mol) is heated with stirring to 55° for 2.5 hours. The reaction mixture is then cooled to 20° and hexane (100 ml) is added. Methanesulfonyl chloride (33.49 g, 0.293 mol) is added slowly over 1.75 hours while maintaining an internal reaction temperature of 15-20°. After 2 hours at 20°, additional methanesulfonyl chloride (0.50 g, 0.004 mol) is added and the mixture is stirred for 1 hour. The water (2.50 g) is added, followed 1 hour later by addition of hexane/toluene (80/20; 330 ml) and water (200 ml). The phases are separated and the organic phase is washed with water, with 10% aqueous HCl solution, with water, with 5% aqueous KOH solution, and with three portions of water. The solvent is stripped off to give (R,S)-α-cyano-3-phenoxybenzyl (R)-2-(2-chloro-4-trifluoromethylanilino)-3-methylbutanoate as an amber coloured oil. Yield is 92.1% (based on the weight of the starting butanoic acid).

**EXAMPLE 4**

Following the procedure of Example 3 (R,S)-α-cyano-4-fluoro-3-phenoxybenzyl (R)-2-(2-chloro-4-trifluoromethylanilino)-3-methylbutanoate is prepared from (R)-2-(2-chloro-4-trifluoromethyanilino)-3-methylbutanoic acid, 4-fluoro-3-phenoxybenzaldehyde and sodium cyanide. Yield is 93.03%, based on starting butanoic acid.

**EXAMPLE 5**

Following the procedure of Example 3, replacing at least one of the cyanide (sodium cyanide), the acid acceptor solvent (mixed picolines) and the aprotic solvent (hexane) by a corresponding amount of the cyanide, acid acceptor solvent and aprotic solvent specified in Table A, (R,S)-α-cyano-3-phenoxybenzyl (R)-2-(2-chloro-4-trifluoromethylanilino)-3-methylbutanoate is obtained in the yield given in said Table A.

TABLE A

| Example | Cyanide | Acid Acceptor Solvent | Aprotic solvent | Yield(*) % |
|---|---|---|---|---|
| 5a | NaCN | pyridine | toluene | 86 |
| 5b | NaCN | dimethylaniline | hexane | 71 |
| 5c | NaCN | 5-ethyl-2-methyl-pyridine | hexane | 85 |
| 5d | NaCN | pyridine | hexane/toluene (6:1) | 91 |
| 5e | NaCN | 2,4,6-collidine | toluene | 89 |
| 5f | NaCN | triethylamine | hexane/toluene (6:1) | 64 |
| 5g | NaCN | "mixed" picolines | methylene chloride | 88 |
| 5h | KCN | "mixed" picolines | toluene | 86 |

(*) based on starting butanoic acid.

**Claims**

1. A process for the preparation of an α-cyano ester of formula I

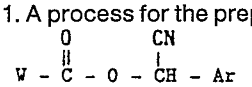 I

wherein W is an optionally substituted cycloalkyl or substituted alkyl group containing up to 24 carbon atoms, and Ar is a substituted aromatic or heteroaromatic group, which process comprises

a) treating a carboxylic acid of formula W-COOH and an aldehyde of formula II

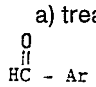 II

with an alkali or alkaline earth metal cyanide in an acid acceptor solvent which is optionally diluted by an aprotic solvent, and

b) treating the resulting mixture with a sulfonyl halide.

2. A process according to Claim 1 wherein Ar is the group of formula VI

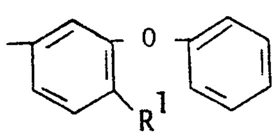 VI

wherein $R^1$ is hydrogen or fluoro.

3. A process according to Claim 2 wherein the acid acceptor solvent is selected from pyridine, picolines or tertiary amines.

4. A process according to Claim 3 wherein the cyanide is an alkali earth metal cyanide.

5. A process according to Claim 4 wherein the cyanide is sodium cyanide.

6. A process according to Claim 5 wherein the acid acceptor solvent is picoline.
7. A process according to Claim 6 wherein the sulfonyl halide is methanesulfonyl chloride.
8. A process according to Claim 7 wherein the aldehyde is 3-phenoxybenzaldehyde.
9. A process according to Claim 8 wherein the carboxylic acid is (R)-2-(2-chloro-4-trifluoromethylanilino)-3-methylbutanoic acid.